(19) Europäisches Patentamt
European Patent Office
Office européen des brevets

(11) EP 1 758 543 B1

(12) EUROPÄISCHE PATENTSCHRIFT

(45) Veröffentlichungstag und Bekanntmachung des Hinweises auf die Patenterteilung:
**05.09.2007 Patentblatt 2007/36**

(21) Anmeldenummer: **05736989.4**

(22) Anmeldetag: **30.04.2005**

(51) Int Cl.:
***A61Q 5/06*** *(2006.01)* ***A61K 8/49*** *(2006.01)*
***A61K 8/41*** *(2006.01)*

(86) Internationale Anmeldenummer:
**PCT/EP2005/004699**

(87) Internationale Veröffentlichungsnummer:
**WO 2005/115316 (08.12.2005 Gazette 2005/49)**

# (54) VERWENDUNG VON SPEZIELLEN FORMYLIERTEN CHINOLINIUMDERIVATEN IN KOMBINATION MIT AMINOSUBSTITUIERTEN DIARYLDERIVATEN UND MINDESTENS EINEM WEITEREN PRIMÄREN ODER SEKUNDÄREN AROMATISCHEN AMIN

USE OF SPECIAL FORMYLATED QUINOLINIUM DERIVATIVES IN CONJUNCTION WITH AMINO-SUBSTITUTED DIARYL DERIVATIVES AND WITH AT LEAST ONE OTHER PRIMARY OR SECONDARY AROMATIC AMINE

UTILISATION DE DERIVES DE QUINOLINIUM FORMYLES SPECIAUX, EN COMBINAISON AVEC DES DERIVES DE DIARYLE SUBSTITUES PAR AMINO ET AU MOINS UNE AUTRE AMINE AROMATIQUE PRIMAIRE OU SECONDAIRE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.05.2004 DE 102004025272**

(43) Veröffentlichungstag der Anmeldung:
**07.03.2007 Patentblatt 2007/10**

(73) Patentinhaber: **Henkel Kommanditgesellschaft auf Aktien**
**40589 Düsseldorf (DE)**

(72) Erfinder:
- **OBERKOBUSCH, Doris**
**40591 Düsseldorf (DE)**
- **HÖFFKES, Horst**
**40595 Düsseldorf (DE)**
- **MOCH, Melanie**
**41542 Dormagen (DE)**

(56) Entgegenhaltungen:
**EP-A- 1 300 132** **DE-A1- 10 148 848**
**DE-A1- 19 859 750**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 758 543 B1

Printed by Jouve, 75001 PARIS (FR)

**Beschreibung**

Die Erfindung betrifft Färbemittel für keratinhaltige Fasern, insbesondere menschliche Haare, die ausgewählte formylierte Chinoliniumderivate in Kombination mit mindestens einem aminosubstituierten Diarylderivat und mindestens einem weiteren primären oder sekundären, aromatischen Amin enthält, sowie ein Verfahren zum Färben von keratinhaltigen Fasern mit diesem Mittel.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere $\alpha$-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe) und Kap. 8, Seiten 264 - 267 (Oxidationsfarbstoffe) sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit OXidationsfarbstoffen lassen sich zwar intensive Färbungen mit guten Echtheitseigenschaften erzielen, die Entwicklung der Farbe geschieht jedoch im Allgemeinen unter Zusatz chemischer Oxidationsmittel wie z.B. $H_2O_2$, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Eine Möglichkeit, Färbungen mit guten Echtheitseigenschaften ohne den Einfluß von zusätzlichen chemischen Oxidationsmitteln zu erzielen ist die Anwendung von sogenannten Oxofärbemitteln. Oxofärbemittel bieten die Möglichkeit keratinhaltige Fasern zu färben, mittels Verwendung einer Kombination aus

Komponente A): Verbindungen, die eine reaktive Carbonylgruppe enthalten, mit
Komponente B): Verbindungen, ausgewählt aus (a) CH-aciden Verbindungen, (b) Verbindungen mit primärer oder sekundärer Aminogruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden enthalten.

Die entsprechende Färbemethode (im folgenden Oxofärbung genannt) wird beispielweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Im Rahmen der Oxofärbung werden als Komponente A reaktive Carbonylverbindungen eingesetzt, die insbesondere nach Reaktion mit einer Komponente B, den eigentlichen Farbstoff im Haar ausbilden. Bevorzugte reaktive

Carbonylverbindungen sind Aldehyde und Ketone, in denen die reaktive Carbonylgruppe entweder als Carbonylgruppe vorliegt oder derart derivatisiert bzw. maskiert ist, daß die Reaktivität des Kohlenstoffatoms der derivatisierten Carbonylgruppe gegenüber den Verbindungen der Komponente B stets vorhanden ist. Diese Derivate sind bevorzugt Additionsverbindungen

a) von Aminen und deren Derivaten unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)

an das Kohlenstoffatom der Carbonylgruppe der reaktiven Carbonylverbindung.

Als hervorragende reaktive Carbonylverbindungen der Komponente A haben sich formylierte Chinoliniumderivate erwiesen, welche durch Reaktion mit Verbindungen der Komponente B intensive Färbungen erzielen. Wichtige anwendungstechnische Kriterien für die erfolgreiche gewerbliche Nutzung von Färbemitteln sind die Egalisierung des Farbaufzugs, die Intensität der Färbung, eine geringe bis nicht vorhandene Hautanfärbung, die Echtheit der Färbung gegenüber Umwelteinflüssen (wie z.B. Waschen, Licht, Reibung, Kaltwellmitteln und Schweiß) und nicht zuletzt die physiologische Verträglichkeit des Färbemittels.

Leider ist die Färbung mit der oben genannten Kombination nicht völlig zufriedenstellend in der Gleichmäßigkeit der Ausfärbung auf unterschiedlich strukturierten keratinhaltigen Fasern. Das Phänomen der unterschiedlich strukturierten keratinhaltigen Fasern tritt verstärkt bei natürlichen Fasern, insbesondere beim Haar, auf, wenn der Haarwuchs Haare oder Haarzonen unterschiedlichen Schädigungsgrades aufweist. Ein Beispiel dafür sind lange Haare, bei denen die Haarspitzen über einen längeren Zeitraum den verschiedensten Umwelteinflüssen ausgesetzt sind und daher in der Regel deutlich stärker geschädigt sind als die relativ frisch nachgewachsenen Haarzonen am Haaransatz.

Aufgabe der vorliegenden Erfindung ist es, Oxofärbemittel für keratinhaltige Fasern, insbesondere menschliche Haare, bereitzustellen, die über die gesamte Länge der Faser gleichmäßig intensive Färbungen liefern. Darüber hinaus sollten die Parameter der Färbungen bezüglich der Farbechtheiten, insbesondere der Waschechtheiten, beibehalten bis verbessert werden.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Es wurde überraschenderweise gefunden, dass diese Aufgabe durch die erfindungsgemäße Oxofarbstoffvorprodukt-Kombination gelöst wird.

Gegenstand der vorliegenden Erfindung ist demgemäß ein Mittel zum Färben keratinhaltiger Fasern, insbesondere menschlicher Haare, welches eine Kombination aus Komponente

(A) mindestens einem formylierten Chinoliniumderivat gemäß Formel I und Derivaten davon

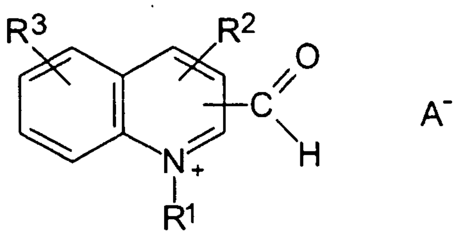

(I)

worin

| | |
|---|---|
| $R^1$ | für eine $(C_1\text{-}C_6)$-Alkylgruppe, $(C_2\text{-}C_6)$-Alkenylgruppe, Arylgruppe, Aryl-$(C_1\text{-}C_6)$-alkylgruppe oder Heteroaryl-$(C_1\text{-}C_6)$-alkylgruppe steht, |
| $R^2$ und $R^3$ | jeweils unabhängig voneinander ein Wasserstoffatom, ein Halogenatom, eine $(C_1\text{-}C_6)$-Alkylgruppe, eine $(C_1\text{-}C_6)$-Alkoxygruppe, Hydroxy-$(C_1\text{-}C_6)$-alkoxygruppe, Hydroxygruppe, Nitrogruppe, Arylgruppe, Trifluormethylgruppe, Aminogruppe, die durch $(C_1\text{-}C_6)$-Alkylgruppen substituiert sein kann, oder $(C_1\text{-}C_6)$-Acylgruppe bedeuten, |
| $A^-$ | ein physiologisch verträgliches Anion oder N-Oxid des Chinolins bedeutet, |

(B1) mindestens ein aminosubstituiertes Diarylderivat oder dessen physiologisch verträgliches Salz mit einer organischen oder anorganischen Säure gemäß Formel II

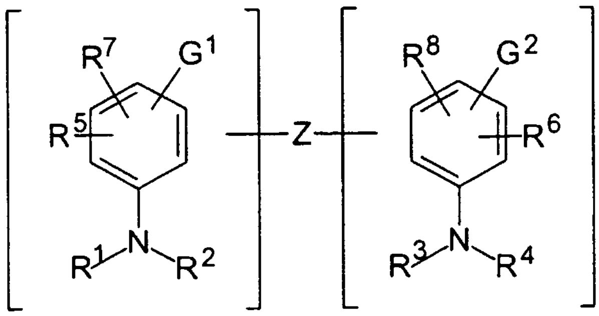

(II)

worin

| | |
|---|---|
| $R^1$, $R^2$, $R^3$ und $R^4$ | unabhängig voneinander eine Bindung zur Verbrückung Z, ein Wasserstoffatom, eine $(C_1-C_6)$-Alkylgruppe, eine $(C_2-C_6)$-Alkenylgruppe, eine gegebenenfalls substituierte Arylgruppe, eine $(C_1-C_6)$-Alkoxy-$(C_1-C_6)$-alkylgruppe, eine $(C_1-C_6)$-Hydroxyalkylgruppe oder eine Aryl-$(C_1-C_6)$-alkylgruppe, bedeuten, wobei auch jeweils die Reste $R^1$ und $R^2$ und/oder $R^3$ und $R^4$ zusammen mit dem Stickstoffatom einen 5- oder 6-gliedrigen Ring bilden können, |
| $R^5$, $R^6$, $R^7$ und $R^8$ | stehen unabhängig voneinander für ein Wasserstoffatom, ein Halogenatom, eine $(C_1-C_6)$-Alkylgruppe, eine $(C_1-C_6)$-Alkoxygruppe, eine $(C_1-C_6)$-Hydroxyalkoxygruppe, eine $(C_2-C_6)$-Polyhydroxyalkylgruppe, eine $(C_1-C_6)$-Alkoxy-$(C_1-C_6)$-alkylgruppe, eine Hydroxy-$(C_1-C_6)$-alkoxygruppe, eine Hydroxygruppe, eine Cyanogruppe, eine Nitrogruppe, eine Carboxygruppe, eine Sulfonsäuregruppe, eine Sulfamoylgruppe, eine gegebenenfalls substituierte Arylazogruppe oder eine Gruppe $R^IR^{II}N-(CH_2)_n-$, worin $R^I$ und $R^{II}$ stehen unabhängig voneinander für ein Wasserstoffatom, eine $(C_1-C_6)$-Alkylgruppe, eine $(C_2-C_6)$-Alkenylgruppe, eine gegebenenfalls substituierte Arylgruppe, eine $(C_1-C_6)$-Alkoxy-$(C_1-C_6)$-alkylgruppe, eine $(C_1-C_6)$-Hydroxyalkylgruppe oder eine Aryl-$(C_1-C_6)$-alkylgruppe, wobei $R^I$ und $R^{II}$ zusammen mit dem Stickstoffatom einen 5- oder 6-gliedrigen Heterozyklus bilden kann, und n steht für eine Zahl 0, 1, 2, 3 oder 4, |
| $G^1$ und $G^2$ | unabhängig voneinander für eine Hydroxygruppe oder eine Gruppe $-NR^9R^{10}$ stehen, worin $R^9$ und $R^{10}$ unabhängig voneinander ein Wasserstoffatom, eine $(C_1-C_6)$-Alkylgruppe, eine $(C_1-C_6)$-Hydroxyalkylgruppe oder eine Bindung zur Verbrückung Z bedeuten, |
| Z | steht für eine direkte Bindung, eine Gruppe NR''', worin R''' ein Wasserstoffatom oder eine $(C_1-C_6)$-Alkylgruppe bedeutet, ein Schwefelatom, eine Carbonylgruppe, eine Vinylengruppe, eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein können und eventuell durch einen oder mehrere Hydroxyl- oder $C_1$-bis $C_8$-Alkoxyreste substituiert sein können, |

mit den Maßgaben, dass die Verbindungen der Formel II nur eine Verbrückung Z pro Molekül enthalten und die Verbindungen der Formel II mindestens eine Aminogruppe enthalten, die mindestens ein Wasserstoffatom trägt und
(B2) mindestens ein von (B1) verschiedenes primäres oder sekundäres, aromatisches Amin oder dessen physiologisch verträgliches Salz mit einer organischen oder anorganischen Säure

enthält.

Unter Derivaten der Verbindungen gemäß Formel I sind im Sinne der Erfindung Additionsverbindungen

a) von Aminen und deren Derivaten unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)

an das Kohlenstoffatom der Carbonylgruppe der reaktiven Carbonylverbindung zu verstehen. Insbesondere sind Hydrate als Derivate gemäß c) bevorzugt.

Beispiele für die als Substituenten im Rahmen dieser Anmeldung genannten $(C_1-C_6)$-Alkylreste sind die Gruppen Methyl, Ethyl, Propyl, Isopropyl, n-Butyl, tert-Butyl, n-Pentyl, neo-Pentyl und n-Hexyl. Ethyl und Methyl sind bevorzugte Alkylreste. Beispiele für bevorzugte $(C_2-C_6)$-Alkenylreste sind Vinyl, Allyl und Butenyl. Erfindungsgemäß bevorzugte $(C_1-C_6)$-Alkoxyreste sind beispielsweise eine Methoxy- oder eine Ethoxygruppe. Weiterhin können als bevorzugte Beispiele für eine $(C_1-C_6)$-Hydroxyalkylgruppe eine Hydroxymethyl-, eine 2-Hydroxyethyl-, eine 2-Hydroxypropyl, eine 3-Hydroxypropyl- oder eine 4-Hydroxybutylgruppe genannt werden. Eine 2-Hydroxyethylgruppe ist besonders bevorzugt. Als bevorzugte Beispiele für eine $(C_1-C_6)$-Alkoxy-$(C_1-C_6)$-alkylgruppe können eine 2-Methoxyethyl-, eine 2-Ethoxyethyl-, eine 2-Methoxypropyl, eine 3-Methoxypropyl- oder eine 4-Methoxybutylgruppe genannt werden.

Beispiele für eine bevorzugte $(C_2-C_6)$-Polyhydroxyalkylgruppe sind die 1,2-Dihydroxyethylgruppe und die 2,3-Dihydroxypropylgruppe. Beispiele für eine $(C_1-C_6)$-Acylgruppe sind Acetyl, Propanoyl und Butanoyl. Bevorzugte Hydroxy-$(C_1-C_6)$-alkoxygruppen sind 2-Hydroxyethoxy, 2-Hydroxypropoxy und 3-Hydroxypropoxy. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Bevorzugte Aryl-$(C_1-C_6)$-alkylgruppen sind Benzyl, 2-Phenylethyl, 5-Phenylpropyl, wobei Benzyl besonders bevorzugt ist. Beispiele für Halogenatome sind F-, Cl-, oder Br-Atome, wobei Cl-Atome ganz besonders bevorzugt sind. Die weiteren verwendeten Begriffe leiten sich erfindungsgemäß von den hier gegebenen Definitionen ab.

Bevorzugte Verbindungen gemäß Formel I sind solche, worin die Formylgruppe -CHO in 2- oder 4-Position gebunden ist.

$A^-$ steht in Formel I bevorzugt für Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Chinolins, besonders bevorzugt für ein p-Toluolsulfonat-, Methansulfonat-, Trifluormethansulfonat-, Methylsulfat- oder Benzolsulfonat-Ion. Das bevorzugte Anion ist das p-Toluolsulfonat-Ion.

$R^1$ steht in Formel I bevorzugt für eine $(C_1-C_6)$-Alkylgruppe.

Die Verbindungen gemäß Formel I werden besonders bevorzugt ausgewählt aus der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylchinolinium und 2-Formyl-1-methylchinolinium. 4-Formyl-1-methyl-chinolinium p-Toluolsulfonat und 2-Formyl-1-methyl-chinolinium p-Toluolsulfonat sind ganz besonders bevorzugte Verbindungen gemäß Formel I.

Die Verbindungen mit der Formel I werden üblicherweise in einer Menge von jeweils 0,03 bis 65 mmol, insbesondere 1 bis 40 mmol, jeweils bezogen auf 100 g des gesamten Färbemittels, eingesetzt.

Es ist bevorzugt, dass gemäß Formel II gilt $R^1=R^3$, $R^2=R^4$, $G^1=G^2$, $R^5=R^7$ und $R^6=R^8$. Gemäß dieser Ausführungsform ist es bevorzugt, dass beide Benzolkerne gemäß Formel II das gleiche Substitutionsmuster besitzen und die Verbindungen gemäß Formel II somit symmetrisch sind.

Die Verbindungen der Komponente (B1) gemäß Formel II werden bevorzugt ausgewählt aus der Gruppe, die gebildet wird aus 4,4'-Diaminostilben, 4,4'-Diaminostilben-2,2'-disulfonsäure-mono- oder -di-Na-Salz, 4-Amino-4'-dimethylaminostilben, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, N,N-Bis-[2-(4-aminophenoxy)-ethyl]-methylamin, N-Phenyl-1,4-phenylendiamin, Bis-(5-amino-2-hydroxyphenyl)-methan, N,N'-Bis-(2-hydroxyethyl)-N,N'-bis-(4-aminophenyl)-1,3-diamino-propan-2-ol, N,N'-Bis-(2-hydroxyethyl)-N,N'-bis-(4-aminophenyl)-ethylendiamin, N,N'-Bis-(4-aminophenyl)-tetramethylendiamin, N,N'-Bis-(2-hydroxyethyl)-N,N'-bis-(4-aminophenyl)-tetramethylendiamin, N,N'-Bis-(4-methylaminophenyl)-tetramethylendiamin, N,N'-Diethyl-N,N'-bis-(4-amino-3-methylphenyl)-ethylendiamin, N,N'-Bis-(4-aminophenyl)-1,4-diazacycloheptan, N,N'-Bis-(2-hydroxy-5-aminobenzyl)-piperazin, 1,3-Bis-(2,4-Diaminophenoxy)-propan und die physiologisch verträglichen Salze dieser Verbindungen.

Die Verbindungen gemäß Formel II werden besonders bevorzugt ausgewählt aus der Gruppe, die gebildet wird, aus 4, 4'-Diaminodiphenylamin, N,N'-Bis-(2-hydroxyethyl)-N,N'-bis-(4-aminophenyl)-1,3-diamino-propan-2-ol, N,N'-Bis-(4-aminophenyl)-1,4-diazacycloheptan, Bis-(5-amino-2-hydroxyphenyl)-methan, 1,3-Bis-(2,4-diaminophenoxy)-propan, 1,8-Bis-(2,5-diaminophenoxy)-3,6-dioxaoctan, sowie den physiologisch verträglichen Salzen dieser Verbindungen.

Unter primären bzw. sekundären aromatischen Aminen sind solche Verbindungen zu verstehen, welche das Stickstoffatom einer primären bzw. sekundären Aminogruppe direkt an ein aromatisches System gebunden haben. Diese aromatischen Systeme können sowohl carbozyklisch als auch heterozyklisch sein. Bevorzugt werden die erfindungsgemäßen Komponenten gemäß (B2) ausgewählt aus Verbindungen der Formel III

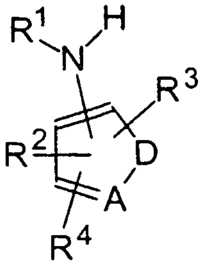

(III)

worin

| | |
|---|---|
| A | für eine Gruppe C-$R^5$, worin $R^5$ ein Wasserstoffatom oder eine Bindung zu einem der Reste $R^2$, $R^3$ oder $R^4$ bedeutet, oder ein Stickstoffatom steht, |
| D | für eine gegebenenfalls substituierte Vinylengruppe, eine Gruppe -N=C$R^6$-, worin $R^6$ ein Wasserstoffatom oder eine Bindung zu einem der Reste $R^2$, $R^3$ oder $R^4$ bedeutet, oder eine Gruppe N-$R^7$ steht, worin $R^7$ eine ($C_1$-$C_6$)-Alkylgruppe, eine ($C_2$-$C_6$)-Alkenylgruppe, eine ($C_1$-$C_6$)-Hydroxyalkylgruppe, eine ($C_2$-$C_6$)-Polyhydroxyalkylgruppe oder eine Aryl-($C_1$-$C_6$)-alkylgruppe bedeutet, |
| $R^1$ | ein Wasserstoffatom, eine ($C_1$-$C_6$)-Alkylgruppe, eine ($C_2$-$C_6$)-Alkenylgruppe, eine ($C_1$-$C_6$)-Hydroxyalkylgruppe, eine ($C_2$-$C_6$)-Polyhydroxyalkylgruppe oder eine Aryl-($C_1$-$C_6$)-alkylgruppe bedeutet, wobei $R^1$ zusammen mit dem Restmolekül einen annelierten 5- oder 6-gliederigen Ring bilden kann, |
| $R^2$, $R^3$ und $R^4$ | unabhängig voneinander ein Wasserstoffatom, ein Halogenatom, eine ($C_1$-$C_6$)-Alkylgruppe, eine ($C_1$-$C_6$)-Alkoxygruppe, eine ($C_1$-$C_6$)-Hydroxyalkoxygruppe, eine ($C_2$-$C_6$)-Polyhydroxyalkylgruppe, eine ($C_1$-$C_6$)-Alkoxy-($C_1$-$C_6$)-alkylgruppe, eine Hydroxy-($C_1$-$C_6$)-alkoxygruppe, eine Hydroxygruppe, eine Cyanogruppe, eine Nitrogruppe, eine Carboxygruppe, eine Sulfonsäuregruppe, eine Sulfamoylgruppe, eine gegebenenfalls substituierte Arylazogruppe oder eine Gruppe $R^IR^{II}N$-$(CH_2)_n$-, <br> worin $R^I$ und $R^{II}$ stehen unabhängig voneinander für ein Wasserstoffatom, eine ($C_1$-$C_6$)-Alkylgruppe, eine ($C_2$-$C_6$)-Alkenylgruppe, eine gegebenenfalls substituierte Arylgruppe, eine ($C_1$-$C_6$)-Alkoxy-($C_1$-$C_6$)-alkylgruppe, eine ($C_1$-$C_6$)-Hydroxyalkylgruppe oder eine Aryl-($C_1$-$C_6$)-alkylgruppe, wobei $R^I$ und $R^{II}$ zusammen mit dem Stickstoffatom einen 5- oder 6-gliedrigen Heterozyklus bilden kann, und n steht für eine Zahl 0, 1, 2, 3 oder 4, <br> bedeuten, wobei auch zwei der Reste $R^2$, $R^3$ und $R^4$ zusammen einen anellierten 5- oder 6-gliedrigen, carbozyklischen oder heterozyklischen, aliphatischen oder aromatischen Ring bilden können |

oder deren physiologisch verträglichen Salze mit anorganischen oder organischen Säuren.

Es ist besonders bevorzugt, die Substituenten A und D gemäß Formel III in folgenden Kombinationen auszuwählen:

A = Stickstoffatom und D = N-$R^7$
A = Stickstoffatom und D = gegebenenfalls substituiertes Vinylen
A = Stickstoffatom und D = -N=C$R^6$-
A = C-$R^5$ und D = gegebenenfalls substituiertes Vinylen.

Ferner ist es besonders bevorzugt, wenn mindestens ein Rest aus $R^2$, $R^3$ und $R^4$ für eine Hydroxygruppe, eine ($C_1$-$C_6$)-Alkoxygruppe oder eine Gruppe $R^IR^{II}N$-$(CH_2)_n$-,
worin $R^I$ und $R^{II}$ wie in Formel III definiert sind und n = 0 ist, steht.

Wenn D in Formel III für eine substituierte Vinylengruppe steht, so ist diese Vinylengruppe bevorzugt mit mindestens einem der Reste $R^2$, $R^3$ oder $R^4$ gemäß Formel III substituiert.

Die primären und sekundären aromatischen Amine werden besonders bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 2-(2,5-Diaminophenyl)-ethanol, 2-(2,4-Diaminophenoxy)ethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Amino-2,6-dichlorphenol, 4-Methylaminophenol, 4-Aminochinaldin, 5-Amino-2-methylphenol,

4-Amino-3-methylphenol, 2-Methyl-5-(2-hydroxyethylamino)phenol, 3-Amino-2-chlor-6-methylphenol, 2-Amino-6-chlor-4-nitrophenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5,6-Dihydroxyindolin, 4-Hydroxyindolin, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl) amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlorbenzol (HC Red Nr. 10), 2-(4-Amino-2-nitroanilino)-benzoesäure, 6-Nitro-2,5-diaminopyridin, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol,

Als weitere primäre oder sekundäre, aromatische Amine sind entsprechende stickstoffhaltige Heteroaromaten mit primärer bzw. sekundärer Aminogruppe zu nennen. Diese sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 4,5-Diamino-1-(2-Hydroxyethyl)pyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indolderivate, wie 4-Aminoindol, 5-Aminoindol, 6-Aminoindol, 7-Aminoindol und 5,6-Dihydroxyindol. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Erfindungsgemäß besonders bevorzugt werden die primären bzw. sekundären aromatischen Amine der Komponente (B2) ausgewählt aus der Gruppe, die gebildet wird aus N,N-Bis-(2-Hydroxyethyl)-p-phenylendiamin, 1-(2-Hydroxyethyl)-p-phenylendiamin, 2,5-Diaminotoluol, 2-(2,4-Diaminophenoxy)ethanol, 2-Amino-4-(2-Hydroxyethyl)aminoanisol, 4-Aminophenol, 4-Amino-3-methylphenol, 4-Amino-2,6-Dichlorphenol, 2-Aminophenol, 3-Aminophenol, 5-Amino-2-methylphenol, 3-Amino-2-chlor-6-methylphenol, 2-Amino-6-chlor-4-nitrophenol, 2,4,5,6-Tetraaminopyrimidin, 4-Hydroxy-2,5,6-triaminopyrimidin, 2-Amino-3-hydroxypyridin, 3-Amino-2-methylamino-6-methoxypyridin, 3,5-Diamino-2,6-dimethoxypyridin, 4,5-Diamino-1-(2-hydroxyethyl)pyrazol, sowie den physiologisch verträglichen Salze der genannten Verbindungen.

Die Komponenten B1 und B2 können jeweils in einer Menge von jeweils 0,03 bis 65 mmol, insbesondere 1 bis 40 mmol, jeweils bezogen auf 100 g der gesamten Anwendungsmischung, eingesetzt werden. Die Verbindungen der Formel 1 einerseits und die Verbindungen der Komponenten B1 und B2 andererseits werden bevorzugt in solchen Mengenverhältnissen eingesetzt, daß die Verbindungen der Formel I und die Summe der Zahl der Aminogruppen der Verbindungen der Komponenten B1 und B2 im Verhältnis 2 : 1 bis 1 : 2 stehen.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Auf die Anwesenheit von zusätzlichen chemischen Oxidationsmitteln, z.B. $H_2O_2$, wird bevorzugt in den erfindungsgemäßen Färbemitteln verzichtet. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist $H_2O_2$.

Desweiteren können die erfindungsgemäßen Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

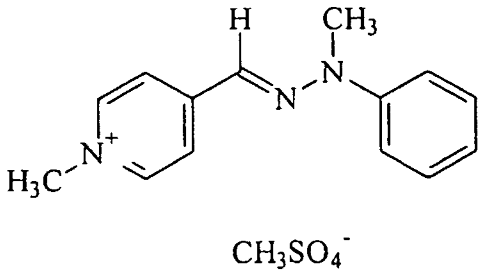

(DZ1)

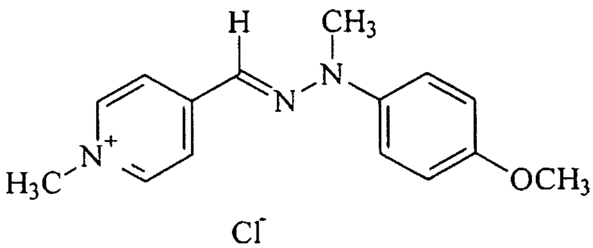

(DZ2)

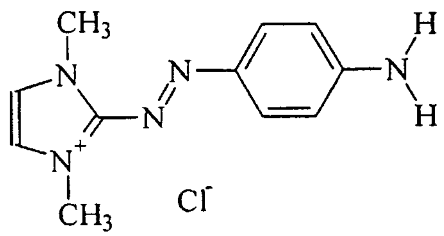

(DZ3)

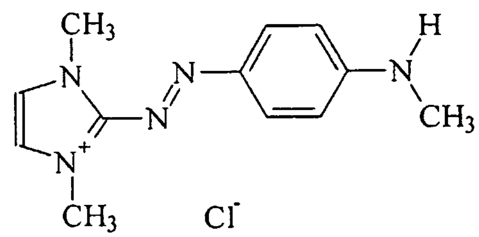

(DZ4)

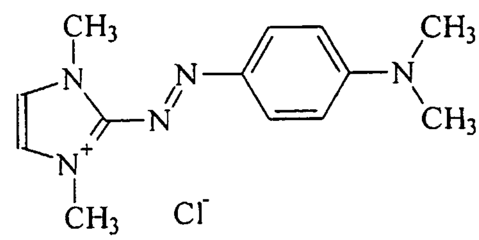

(DZ5)

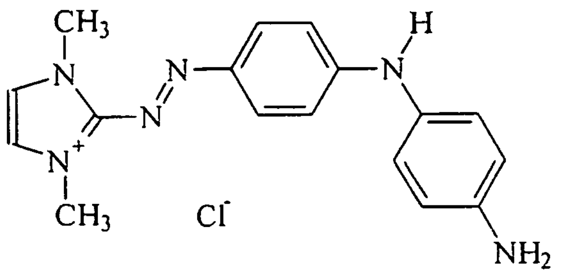

(DZ6)

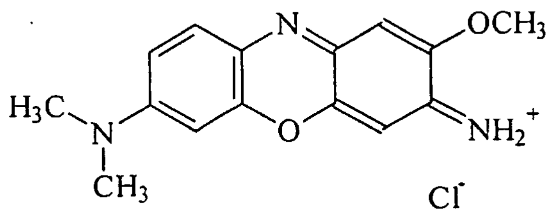

(DZ7)

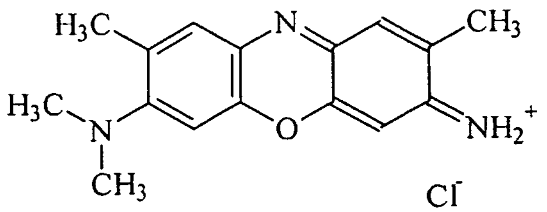

(DZ8)

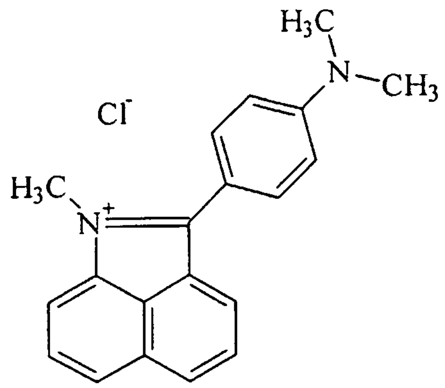

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Färbemittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung

auf dem menschlichen Haar können die erfindungsgemäße Zusammensetzungenüblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind.

Die erfindungsgemäßen Mittel enthalten in einer weiteren Ausführungsform zusätzlich mindestens ein lineares oder verzweigtes, aliphatisches Polyol, bevorzugt in einer Menge von 2 bis 50 Gew.-%, besonders bevorzugt in einer Menge von 5 bis 30 Gew.-%, jeweils bezogen auf das Gewicht des gesamten Färbemittels.

Bevorzugte Vertreter der erfindungsgemäß verwendbaren Polyole werden aus einer Gruppe ausgewählt, die gebildet wird aus $HO-(CH_2CH_2O)_n-H$ mit n = 1 bis 14, Dipropylenglykol, Tripropylenglykol, Glycerin, Diglycerin, 1,2-Butandiol, 1,4-Butandiol, 1,5-Pentandiol, 1,2-Pentandiol, 2-Methyl-2,4-pentandiol, 4-Methyl-2-pentanol, 1,2,6-Hexantriol und 2-Ethyl-1,3-hexandiol. Ein besonders bevorzugtes Polyol ist Ethylenglykol.

Zusätzlich können die erfindungsgemäßen Färbemittel mindestens ein Polymer, ausgewählt aus der Gruppe, die gebildet wird aus anionischen, amphoteren, kationischen und nichtionischen Polymeren, insbesondere aus kationischen und nichtionischen Polymeren. Dabei kann es wiederum bevorzugt sein, wenn die oben genannten Polymertypen von Polysacchariden und dessen Derivaten abgeleitet sind.

Bei den verwendbaren anionischen Polymeren, handelt es sich um ein anionische Polymere, welche Carboxylat- und/oder Sulfonatgruppen und/oder Phosphatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Ein Beispiel für ein anionisches Polymer mit einer Phosphatgruppe ist das unter dem Handelsnamen Structure® Zea vertriebene Polysaccharidderivat (NATIONAL STARCH) (INCI-Bezeichnung: Hydroxypropyl Starch Phosphate).

Weitere verwendbare anionische Polymere, enthalten als alleiniges oder Co-Monomer 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Weitere anionische Copolymere sind Acrylsäure-Acrylamid-Copolymere sowie insbesondere Polyacrylamidcopolymere mit Sulfonsäuregruppen-haltigen Monomeren. Ein solches anionisches Copolymer besteht bevorzugt aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Weiterhin können als Polymere amphotere Polymere eingesetzt werden. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefaßt, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2

104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus

(a) Monomeren mit quartären Ammoniumgruppen der allgemeinen Formel P1,

$$R^1\text{-CH=CR}^2\text{-CO-Z-(C}_nH_{2n})\text{-N}^{(+)}R^3R^4R^5\ A^{(-)} \qquad \text{(P1)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist und

(b) monomeren Carbonsäuren der allgemeinen Formel P2,

$$R^1\text{-CH=CR}^2\text{-COOH} \qquad \text{(P2)}$$

in denen $R^1$ und $R^2$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere der Formel P1 eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres gemäß Formel P1. Als Monomeres der Formel P2 für die genannten Polymerisate wird bevorzugt Acrylsäure verwendet.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert der Mittel eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.
Homopolymere der allgemeinen Formel P3,

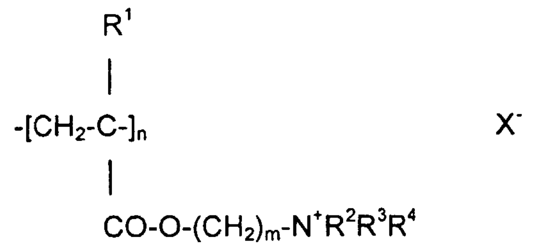

(P3)

in der $R^1$= -H oder $-CH_3$ ist, $R^2$, $R^3$ und $R^4$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel P3 aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gemäß Formel P3 gilt:

- $R^1$ steht für eine Methylgruppe
- $R^2$, $R^3$ und $R^4$ stehen für Methylgruppen
- m hat den Wert 2.

Als physiologisch verträgliches Gegenion $X^-$ gemäß Formel P3 kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel P3 enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar (COGNIS), N-Hance® 3000, N-Hance® 3196, N-Hance® 3205, N-Hance® 3215 (alle AQUALON) und Jaguar® (RHODIA) vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylaminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,

sowie die unter den Bezeichnungen

- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von $5 \cdot 10^5$ bis $5 \cdot 10^6$ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bevorzugt verwendete nichtionogene Polymere werden ausgewählt aus:

- VinylpyrrolidonNinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Cellulose und Derivate davon, insbesondere Celluloseether, wie Hydroxypropylcellulose, Hydroxyethylcellulose und Methylhydroxypropylcellulose, wie sie beispielsweise unter den Warenzeichen Klucel® (INCI-Bezeichnung: Hydroxypropylcellulose), Culminal® (INCI-Bezeichnung: Hydroxypropylcellulose) und Benecel® (INCI-Bezeichnung: Hydroxypropyl Methylcellulose) (alle AQUALON) sowie Natrosol® (AQUALON), insbesondere Natrosol® 250 HR (INCI-Bezeichnung: Hydroxyethylcellulose) und Natrosol® Plus 330 CS (INCI-Bezeichnung: Cetylhydroxyethylcellulose) vertrieben werden.
- Guar und Derivate davon, wie sie beispielsweise unter der Warenzeichen N-Hance® HP-40 (AQUALON) vertrieben werden
- Stärke-Fraktionen und Derivate davon, wie beispielsweise Amylose, Amylopektin und Dextrine, die z.B. unter dem Handelsnamen Amaze® und Structure® Solanance (NATIONAL STARCH) erhältlich sind- Schellack
- Polyvinylpyrrolidone, wie sie beispielsweise unter der Bezeichnung Luviskol® (BASF) vertrieben werden.
- Agar-Agar, Alginate, Xanthan-Gum, Gummi arabicum, Karaya-Gummi, Johannisbrotkernmehl, Leinsamengummen, Dextrane.

Es ist besonders bevorzugt, solche nichtionischen Polymere zu verwenden, welche sich von einem Polysaccharid ableiten, wie Cellulose und seine Derivate, Stärke-Fraktionen und deren Derivate, Guar und seine Derivate.

Die Polymere werden erfindungsgemäß bevorzugt in Mengen von 0,05 bis 5 Gew.-%, bezogen auf die gesamte Zusammensetzung, eingesetzt. Mengen von 0,25 bis 2 Gew.% sind besonders bevorzugt.

Weiterhin können die erfindungsgemäßen Mittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,

- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_8$-$_{18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12-22}$-Fettsäuremono- und -diester von Anlagerungsprodukten von 1 bis 30 Mol Ethylenoxid an Glycerin,
- $C_{8-22}$-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Mitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch

ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden bevorzugt in Konzentrationen von 0,5 bis 30 Gew.-%, bezogen auf das Gewicht der Zusammensetzung, eingesetzt.

Die erfindungsgemäßen Mittel können zusätzlich einen Fettalkohol enthalten. Unter Fettalkoholen sind primäre aliphatische Monoalkohole mit einem aliphatischen, linearen oder verzweigten Kohlenwasserstoffrest mit 6 bis 30 Kohlenstoffatomen und 0 und/oder 1, 2 oder 3 Doppelbindungen steht. Typische Beispiele sind Ca-pronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalko-hol, Petroselinylalkohol, Linolylalkohol, Linolenylalkohol, Elaeostearylalkohol, Arachylalkohol, Gadoleyl-alkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Die erfindungsgemäßen Mittel können zusätzlich einen Ölkörper enthalten. Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, Ester von linearen $C_6$-$C_{22}$-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/oder Guerbetalkoholen, Triglyceride auf Basis $C_6$-$C_{10}$-Fettsäuren, flüssige Mono-/Di/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Weitere Wirk-, Hilfs- und Zusatzstoffe sind beispielsweise

- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,

- Quell- und Penetrationsstoffe wie Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien.

Ein zweiter Gegenstand der Erfindung ist ein Verfahren zum Färben von keratinhaltigen Fasern, insbesondere menschlichen Haaren, in welchem ein Mittel des ersten Gegenstandes der Erfindung auf das Haar aufgebracht wird, und nach einer Einwirkzeit wieder vom Haar abgespült wird.

Die erfindungsgemäßen Komponenten A, B1 und B2 können als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. In der Regel werden die Komponenten gemeinsam mit einem wasserhaltigen, kosmetischen Träger in einer Menge von 100 g auf das Haar aufgebracht, ca. 30 Minuten dort belassen und anschließend ausgespült oder mit einem handelsüblichen Haarshampoo ausgewaschen.

In einer bevorzugten Ausführungsform des erfindungsgemäßen Verfahrens wird das erfindungsgemäße Färbemittel unmittelbar vor der Anwendung aus einer ersten Zusammensetzung, welche die Komponente A enthält, mit einer zweiten Zusammensetzung, enthaltend Komponenten B1 und B2, gemischt.

Es ist bevorzugt, dass die erste Zusammensetzung einen pH-Wert von 1 bis 7, bevorzugt von 3 bis 6, besitzt und die zweite Zusammensetzung auf einen pH-Wert von 2 bis 11 eingestellt ist. Der pH-Wert der gebrauchsfertigen Anwendungsmischungen liegt üblicherweise zwischen 2 und 11, vorzugsweise zwischen 6 und 10. Wenn beide Zusammensetzungen flüssig sind, ist das Mischverhalten optimal, wenn die erste Zusammensetzung eine Viskosität von eine Viskosität von 50 bis 100000 mPa˙s besitzt und die zweite Zusammensetzung eine Viskosität von 15000 bis 150000 mPa˙s hat. Die Anwendungsmischung erhält eine Viskosität von 5000 bis 50000 mPa˙s, lässt sich leicht auftragen und erlaubt eine rasche Penetration der farbstoffbildenden Komponenten in das Haar. Alle Viskositäten werden bei 20°C mit einem Rotationsviskosimeter mit Spindel Nr. 4 bestimmt.

Die fakultativ enthaltenen Ammonium- oder Metallsalze können allein oder im Gemisch mit den weiteren Komponenten zugesetzt werden. Auch eine Vorbehandlung der Fasern mit der Salzlösung ist möglich.

Enthält das Haarfärbemittel neben den Komponenten A, B1 und B2 zusätzlich als chemisches Oxidationsmittel Wasserstoffperoxid oder ein wasserstoffperoxidhaltiges Oxidationsmittelgemisch, gegebenenfalls in Kombination mit anorganischen Peroxoverbindungen wie Persulfaten oder Peroxodisulfaten, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationsmittel kann unmittelbar vor der Anwendung mit dem Haarfärbemittel gemischt und die Mischung auf das Haar aufgebracht werden. Werden die Verbindungen der Komponente A und die Verbindungen der Komponenten B1 und B2 in einem zweistufigen Verfahren nacheinander auf das Haar appliziert, ist das Oxidationsmittel in einer der beiden Verfahrensstufen zusammen mit der entsprechenden farbgebenden Komponente anzuwenden. Zu diesem Zweck kann das Oxidationsmittel mit der Komponente A in einem Container oder getrennt konfektioniert werden.

Die erfindungsgemäßen Komponenten A, B1 und B2 können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

Beispiele

Alle Mengenangeben sind, soweit nicht anders gekennzeichnet, Gewichtsprozent (Gew.-%). Folgende Handelsprodukte wurden als Rohstoffe verwendet:

| | |
|---|---|
| Hydrenol® D | $C_{16}$-$C_{18}$-Fettalkohol (INCI-Bezeichnung: Cetearyl alcohol) (Cognis Deutschland) |
| Lorol® techn. | $C_{12}$-$C_{18}$-Fettalkohol (INCI-Bezeichnung: Coconut alcohol) (Cognis Deutschland) |
| Eumulgin® B 2 | Cetylstearylalkohol mit ca. 20 EO-Einheiten (INCI-Bezeichnung: Ceteareth-20) (Cognis Deutschland) |
| Turpinal® SL | 1-Hydroxyethan-1,1-diphosphonsäure (INCI-Bezeichnung: Etidronic Acid, Aqua (Water)) (Solutia) |
| Natrosol® 250HR | Hydroxyethylcellulose (INCI-Bezeichnung: Hydroxyethylcellulose) (Hercules) |

Es wurden folgende Komponenten nach bekanntem Verfahren hergestellt:

Tabelle 1: Farbcremes

| | H1 | H2 | H3 | H4 | H5 | H6 |
|---|---|---|---|---|---|---|
| Hydrenol® D | 7,50 | 7,50 | 7,50 | 7,50 | 7,50 | 7,50 |
| Lorol® techn. | 2,50 | 2,50 | 2,50 | 2,50 | 2,50 | 2,50 |
| Eumulgin® B2 | 2,00 | 2,00 | 2,00 | 2,00 | 2,00 | 2,00 |
| 2-(2-Hydroxyethyl)-p-phenylendiamin | - | - | - | - | 2,00 | 1,00 |
| 2-(2,4-Diaminophenoxy)ethanol | - | 1,93 | 0,96 | - | - | - |
| 1,3-Bis(2,4-diaminophenoxy)-propan 4 HCl | 3,76 | - | 1,88 | - | - | - |
| 2,10-Bis(2,5-diaminophenyl)-1,4.7,10-tetraoxadecan4 HCl | - | - | - | 4,21 | - | 2,11 |
| Turpinal® SL | 0,20 | 0,20 | 0,20 | 0,20 | 0,20 | 0,20 |
| Natronwasserglas 40/42 | 0,50 | 0,50 | 0,50 | 0,50 | 0,50 | 0,50 |
| Parfum | 0,20 | 0,20 | 0,20 | 0,20 | 0,20 | 0,20 |
| Monoethanolamin | | | ad pH 9,4 | | | |
| Wasser, dest. | | | ad 100 | | | |

Tabelle 2: Gel

| | Gel |
|---|---|
| 4-Formyl-1-methyl-chinolinium-p-toluolsulfonat | 2,75 |
| Ethylenglykol | 15,00 |
| Konservierungsmittel | 1,20 |
| Natrosol® 250HR | 1,00 |
| Parfum | 0,10 |
| Wasser, dest. | ad 100 |

Ausfärbung:

Die Gelkomponente wird im Gew.-Verhältnis 1:1 mit einer der Farbcremes gemischt und auf Egalisiersträhnen appliziert (2 g Farbmischung pro 1g Haar). Nach einer Einwirkzeit von 30 min bei 32°C wird die Farbmischung ausgespült und mit Shampoo gewaschen.

Pro Farbmischung wurden 4 Egalisiersträhnen eingefärbt.

Herstellung der Egalisiersträhnen:

Egalisiersträhnen werden hergestellt, indem 18 cm lange Strähnen mit 80% Grauanteil (Fa Kerling) in der Mitte abgebunden und im oberen Teil einmal mit Poly Blonde Ultra (Fa. Schwarzkopf & Henkel) blondiert, im unteren Teil je 2x im Wechsel mit Poly Lock normale Welle (Fa. Schwarzkopf & Henkel) dauergewellt und mit Poly Blonde Ultra (Fa. Schwarzkopf & Henkel) blondiert werden. Auf diese Weise werden unterschiedlich geschädigte Bereiche im Haar erhalten, die es ermöglichen, die Egalisierung, d.h. die Gleichmäßigkeit der Färbung der unterschiedlich stark geschädigten Haarpartien, zu prüfen.

Bestimmung der Egalisierung:

Die Strähnen wurden direkt nach der Färbung und nach sechsmaligem Shampoonieren im oberen und unteren Teil farbmetrisch mit dem Gerät Dataflash SF450 der Firma Datacolor vermessen und die Lab-Werte bestimmt. An jeder Strähne wurden 6 Messungen am oberen Teil und 6 Messungen am unteren Teil der Strähne durchgeführt und die Messwerte je Strähnenteil pro Strähne gemittelt. Die aufgeführten CIELab-Koordinaten sind ein Maß für L (Helligkeit), a (Farbe Rot-Grün-Anteil), b (Farbe Gelb-Blau-Anteil).Der Farbabstand $\Delta E$ wurde gemäß folgender Farbabstandsformel berechnet:

$$\Delta E = \sqrt{\left((\Delta L)^2 + (\Delta a)^2 + (\Delta b)^2\right)}$$

Der Farbabstand ist ein Maß für die Farbveränderung. Die Werte ΔL, Δa und Δb werden wie folgt berechnet:

$$\Delta L = L_{\text{oberer Strähnenteil}} - L_{\text{unterer Strähnenteil}}$$

$$\Delta a = a_{\text{oberer Strähnenteil}} - a_{\text{unterer Strähnenteil}}$$

$$\Delta b = b_{\text{oberer Strähnenteil}} - b_{\text{unterer Strähnenteil}}$$

Die Werte der jeweiligen Ausfärbungen werden nachfolgend zusammengefasst.

Haarfärbemischung H1: Gel + Farbcreme A (Vergleich):
Egalisierung nach 6 Wäschen : ΔE = 7,5

Haarfärbemischung H2: Gel + Farbcreme B (Vergleich):
Egalisierung nach 6 Wäschen : ΔE = 6,7

Haarfärbemischung H3: Gel + Farbcreme C (erfindungsgemäß):
Egalisierung nach 6 Wäschen: ΔE = 1,3

Haarfärbemischung H4: Gel + Farbcreme A (Vergleich):
Egalisierung nach 6 Wäschen : ΔE = 8,3

Haarfärbemischung H5: Gel + Farbcreme B (Vergleich):
Egalisierung nach 6 Wäschen : ΔE = 10,9

Haarfärbemischung H6: Gel + Farbcreme C (erfindungsgemäß):
Egalisierung nach 6 Wäschen: ΔE = 6,6

Nach 6 Wäschen wurde die Egalisierung von Oxofärbungen mit Formylchinoliniumderivaten durch die erfindungsgemäße Kombination von aminosubstituiertem Diarylderivat mit einem primären oder sekundären, aromatischen Amin verbessert.

Prüfung der Hautanfärbung

An drei Probanden werden je 30g der Haarfärbemischungen H1, H2 und H3 angewendet. Zu diesem Zweck wird das Kopfhaar jedes Probanden in drei gleichgroße Flächen aufgeteilt und pro Fläche eine der Haarfärbemischungen aufgetragen. Die Mischungen wurden 30 Minuten auf dem Haar belassen und anschließend mit Wasser ausgespült. Das Haar wurde shampooniert und getrocknet.
Ein analoges Experiment wurde mit drei Probanden und den Haarfärbemischungen H4, H5 und H6 durchgeführt.
Die Kopfhautpartie mit Kontakt zur erfindungsgemäßen Haarfärbemischung H3 bzw. H6 wies bei allen drei Probanden eine deutlich geringere Hautanfärbung auf, als die restlichen Kopfhautpartien.

## Patentansprüche

1. Mittel zum Färben keratinhaltiger Fasern, insbesondere menschlicher Haare, welches eine Kombination aus den Komponenten

(A) mindestens einem formylierten Chinoliniumderivat gemäß Formel I und Derivaten davon

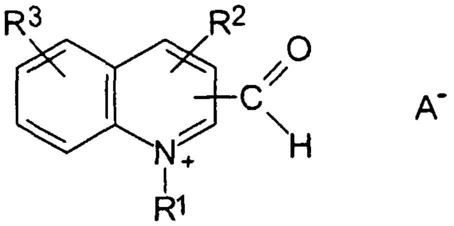

(I)

worin

$R^1$ für eine ($C_1$-$C_6$)-Alkylgruppe, ($C_2$-$C_6$)-Alkenylgruppe, Arylgruppe, Aryl-($C_1$-$C_6$)-alkylgruppe oder Heteroaryl-($C_1$-$C_6$)-alkylgruppe steht,
$R^2$ und $R^3$ jeweils unabhängig voneinander ein Wasserstoffatom, ein Halogenatom, eine ($C_1$-$C_6$)-Alkylgruppe, eine ($C_1$-$C_6$)-Alkoxygruppe, Hydroxy-($C_1$-$C_6$)-alkoxygruppe, Hydroxygruppe, Nitrogruppe, Arylgruppe, Trifluormethylgruppe, Aminogruppe, die durch ($C_1$-$C_6$)-Alkylgruppen substituiert sein kann, oder ($C_1$-$C_6$)-Acylgruppe bedeuten,
$A^-$ ein physiologisch verträgliches Anion oder N-Oxid des Chinolins bedeutet,

(B1) mindestens ein aminosubstituiertes Diarylderivat oder dessen physiologisch verträgliches Salz mit einer organischen oder anorganischen Säure gemäß Formel II

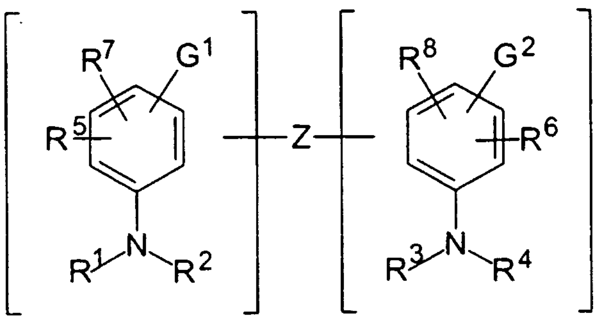

(II)

worin

$R^1$, $R^2$, $R^3$ und $R^4$ unabhängig voneinander eine Bindung zur Verbrückung Z, ein Wasserstoffatom, eine ($C_1$-$C_6$)-Alkylgruppe, eine ($C_2$-$C_6$)-Alkenylgruppe, eine gegebenenfalls substituierte Arylgruppe, eine ($C_1$-$C_6$)-Alkoxy-($C_1$-$C_6$)-alkylgruppe, eine ($C_1$-$C_6$)-Hydroxyalkylgruppe oder eine Aryl-($C_1$-$C_6$)-alkylgruppe, bedeuten, wobei auch jeweils die Reste $R^1$ und $R^2$ und/oder $R^3$ und $R^4$ zusammen mit dem Stickstoffatom einen 5- oder 6-gliedrigen Ring bilden können,
$R^5$, $R^6$, $R^7$ und $R^8$ stehen unabhängig voneinander für ein Wasserstoffatom, ein Halogenatom, eine ($C_1$-$C_6$)-Alkylgruppe, eine ($C_1$-$C_6$)-Alkoxygruppe, eine ($C_1$-$C_6$)-Hydroxyalkoxygruppe, eine ($C_2$-$C_6$)-Polyhydroxyalkylgruppe, eine ($C_1$-$C_6$)-Alkoxy-($C_1$-$C_6$)-alkylgruppe, eine Hydroxy-($C_1$-$C_6$)-alkoxygruppe, eine Hydroxygruppe, eine Cyanogruppe, eine Nitrogruppe, eine Carboxygruppe, eine Sulfonsäuregruppe, eine Sulfamoylgruppe, eine gegebenenfalls substituierte Arylazogruppe oder eine Gruppe $R^IR^{II}N$-$(CH_2)_n$-, worin $R^I$ und $R^{II}$ stehen unabhängig voneinander für ein Wasserstoffatom, eine ($C_1$-$C_6$)-Alkylgruppe, eine ($C_2$-$C_6$)-Alkenylgruppe, eine gegebenenfalls substituierte Arylgruppe, eine ($C_1$-$C_6$)-Alkoxy-($C_1$-$C_6$)-alkylgruppe, eine ($C_1$-$C_6$)-Hydroxyalkylgruppe oder eine Aryl-($C_1$-$C_6$)-alkylgruppe, wobei $R^I$ und $R^{II}$ zusammen mit dem Stickstoffatom einen 5- oder 6-gliedrigen Heterozyklus bilden kann, und n steht für eine Zahl 0, 1, 2, 3 oder 4,
$G^1$ und $G^2$ unabhängig voneinander für eine Hydroxygruppe oder eine Gruppe -$NR^9R^{10}$ stehen, worin $R^9$ und $R^{10}$ unabhängig voneinander ein Wasserstoffatom, eine ($C_1$-$C_6$)-Alkylgruppe, eine ($C_1$-$C_6$)-Hydroxyalkylgruppe oder eine Bindung zur Verbrückung Z bedeuten,

Z steht für eine direkte Bindung, eine Gruppe NR''', worin R''' für ein Wasserstoffatom oder eine $(C_1-C_6)$-Alkylgruppe steht, ein Schwefelatom, eine Carbonylgruppe, eine Vinylengruppe, eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein können und eventuell durch einen oder mehrere Hydroxyl- oder $C_1$- bis $C_8$-Alkoxyreste substituiert sein können,

mit den Maßgaben, dass die Verbindungen der Formel II nur eine Verbrückung Z pro Molekül enthalten und die Verbindungen der Formel II mindestens eine Aminogruppe enthalten, die mindestens ein Wasserstoffatom trägt, und
(B2) mindestens ein von (B1) verschiedenes primäres oder sekundäres, aromatisches Amin oder physiologisch verträgliches Salz mit einer organischen oder anorganischen Säure

enthält.

2. Mittel nach Anspruch 1, **dadurch gekennzeichnet, daß** in Formel I die Gruppe - CHO in 2- oder 4-Position gebunden ist.

3. Mittel nach einem der Ansprüche 1 oder 2, **dadurch gekennzeichnet, daß** die Verbindungen gemäß Formel 1 ausgewählt werden aus der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylchinolinium und 2-Formyl-1-methylchinolinium.

4. Mittel nach einem der Ansprüche 1 bis 3, **dadurch gekennzeichnet, daß** die Komponente A als Hydrat vorliegt.

5. Mittel nach einem der Ansprüche 1 bis 4, **dadurch gekennzeichnet, daß** die Verbindungen der Komponente B1 aus der Gruppe ausgewählt werden, die gebildet wird aus 4, 4'-Diaminodiphenylamin, N,N'-Bis-(2-hydroxyethyl)-N, N'-bis-(4-aminophenyl)-1,3-diamino-propan-2-ol, N,N'-Bis-(4-aminophenyl)-1,4-diazacycloheptan, Bis-(5-amino-2-hydroxyphenyl)-methan, 1,3-Bis-(2,4-diaminophenoxy)-propan, 1,8-Bis-(2,5-diaminophenoxy)-3,6-dioxaoctan, sowie den physiologisch verträglichen Salzen dieser Verbindungen.

6. Mittel nach einem der Ansprüche 1 bis 5, **dadurch gekennzeichnet, daß** die Komponente B2 ausgewählt wird aus Verbindungen gemäß Formel III

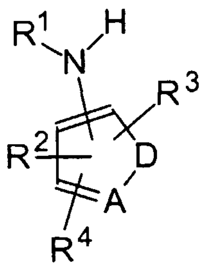

(III)

worin

A für eine Gruppe $C-R^5$, worin $R^5$ ein Wasserstoffatom oder eine Bindung zu einem der Reste $R^2$, $R^3$ oder $R^4$ bedeutet, oder ein Stickstoffatom steht,
D für eine gegebenenfalls substituierte Vinylengruppe, eine Gruppe $-N=CR^6-$, worin $R^6$ ein Wasserstoffatom oder eine Bindung zu einem der Reste $R^2$, $R^3$ oder $R^4$ bedeutet, oder eine Gruppe $N-R^7$ steht, worin $R^7$ eine $(C_1-C_6)$-Alkylgruppe, eine $(C_2-C_6)$-Alkenylgruppe, eine $(C_1-C_6)$-Hydroxyalkylgruppe, eine $(C_2-C_6)$-Polyhydroxyalkylgruppe oder eine Aryl-$(C_1-C_6)$-alkylgruppe bedeutet,
$R^1$ ein Wasserstoffatom, eine $(C_1-C_6)$-Alkylgruppe, eine $(C_2-C_6)$-Alkenylgruppe, eine $(C_1-C_6)$-Hydroxyalkylgruppe, eine $(C_2-C_6)$-Polyhydroxyalkylgruppe, eine Aryl-$(C_1-C_6)$-alkylgruppe bedeutet, wobei $R^1$ zusammen mit dem Restmolekül einen annelierten 5- oder 6-gliederigen Ring bilden kann,
$R^2$ $R^3$ und $R^4$ unabhängig voneinander ein Wasserstoffatom, ein Halogenatom, eine $(C_1-C_6)$-Alkylgruppe, eine

($C_1$-$C_6$)-Alkoxygruppe, eine ($C_1$-$C_6$)-Hydroxyalkoxygruppe, eine ($C_2$-$C_6$)-Polyhydroxyalkylgruppe, eine ($C_1$-$C_6$)-Alkoxy-($C_1$-$C_6$)-alkylgruppe, eine Hydroxy-($C_1$-$C_6$)-alkoxygruppe, eine Hydroxygruppe, eine Cyanogruppe, eine Nitrogruppe, eine Carboxygruppe, eine Sulfonsäuregruppe, eine Sulfamoylgruppe, eine gegebenenfalls substituierte Arylazogruppe, eine Gruppe $R^IR^{II}N$-$(CH_2)_n$-,
worin $R^I$ und $R^{II}$ stehen unabhängig voneinander für ein Wasserstoffatom, eine ($C_1$-$C_6$)-Alkylgruppe, eine ($C_2$-$C_6$)-Alkenylgruppe, eine gegebenenfalls substituierte Arylgruppe, eine ($C_1$-$C_6$)-Alkoxy-($C_1$-$C_6$)-alkylgruppe, eine ($C_1$-$C_6$)-Hydroxyalkylgruppe oder eine Aryl-($C_1$-$C_6$)-alkylgruppe, wobei $R^I$ und $R^{II}$ zusammen mit dem Stickstoffatom einen 5- oder 6-gliedrigen Heterozyklus bilden kann, und n steht für eine Zahl 0, 1, 2, 3 oder 4,

bedeuten, wobei auch zwei der Reste $R^2$, $R^3$ und $R^4$ zusammen einen anellierten 5- oder 6-gliedrigen, carbozyklischen oder heterozyklischen, aliphatischen oder aromatischen Ring bilden können
oder deren physiologisch verträglichen Salze mit anorganischen oder organischen Säuren.

7. Mittel nach einem der Ansprüche 1 bis 5, **dadurch gekennzeichnet, daß** die Verbindungen der Komponente B2 ausgewählt werden aus der Gruppe, bestehend aus Erfindungsgemäß besonders bevorzugt werden die primären bzw. sekundären aromatischen Amine der Komponente (B2) ausgewählt aus der Gruppe, die gebildet wird aus N, N-Bis-(2-Hydroxyethyl)-p-phenylendiamin, 1-(2-Hydroxyethyl)-p-phenylendiamin, 2,5-Diaminotoluol, 2-(2,4-Diaminophenoxy)ethanol, 2-Amino-4-(2-Hydroxyethyl)amino-anisol, 4-Aminophenol, 4-Amino-3-methylphenol, 4-Amino-2,6-Dichlorphenol, 2-Aminophenol, 3-Aminophenol, 5-Amino-2-methylphenol, 3-Amino-2-chlor-6-methylphenol, 2-Amino-6-chlor-4-nitrophenol, 2,4,5,6-Tetraaminopyrimidin, 4-Hydroxy-2,5,6-triaminopyrimidin, 2-Amino-3-hydroxypyridin, 3-Amino-2-methylamino-6-methoxypyridin, 3,5-Diamino-2,6-dimethoxypyridin, 4,5-Diamino-1-(2-hydroxyethyl)pyrazol, sowie den physiologisch verträglichen Salze der genannten Verbindungen.

8. Zusammensetzung, nach einem der Ansprüche 1 bis 7, **dadurch gekennzeichnet**, sie zusätzlich lineare oder verzweigte, aliphatische Polyole enthält.

9. Mittel nach einem der Ansprüche 1 bis 8, **dadurch gekennzeichnet, daß** sie zusätzlich mindestens ein Polymer, ausgewählt aus anionischen, kationischen, nichtionischen und amphoteren Polymeren, enthält.

10. Mittel nach einem der Ansprüche 1 bis 9, **dadurch gekennzeichnet, daß** sie zusätzlich mindestens ein Tensid, ausgewählt aus anionischen, kationischen, nichtionischen, amphoteren und zwitterionischen Tensiden, enthält

11. Mittel nach einem der Ansprüche 1 bis 10, **dadurch gekennzeichnet, daß** sie zusätzlich mindestens einen Fettalkohol mit 6 bis 30 Kohlenstoffatomen enthält.

12. Verfahren zum Färben von keratinhaltigen Fasern, insbesondere menschlichen Haaren, **dadurch gekennzeichnet, daß** ein Mittel gemäß einem der Ansprüche 1 bis 11 auf die Fasern aufgetragen und nach einer Einwirkzeit wieder abgespült wird.

## Claims

1. Composition for colouring keratin-containing fibres, in particular human hair, which comprises a combination of the components

(A) at least one formylated quinolinium derivative according to formula I and derivatives thereof

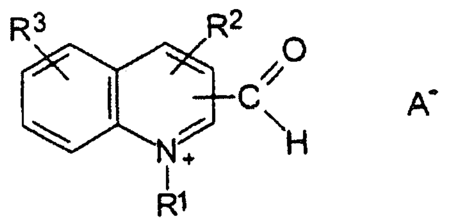

(I)

in which

$R^1$ is a ($C_1$-$C_6$) -alkyl group, ($C_2$-$C_6$)-alkenyl group, aryl group, aryl-($C_1$-$C_6$) -alkyl group or heteroaryl-($C_1$-$C_6$) -alkyl group,
$R^2$ and $R^3$, in each case independently of one another, are a hydrogen atom, a halogen atom, a ($C_1$-$C_6$)-alkyl group, a ($C_1$-$C_6$)-alkoxy group, hydroxy-($C_1$-$C_6$)-alkoxy group, hydroxy group, nitro group, aryl group, trifluoromethyl group, amino group, which may be substituted by ($C_1$-$C_6$)-alkyl groups, or ($C_1$-$C_6$)-acyl group,
A is a physiologically compatible anion or N-oxide of quinoline,

(B1) at least one amino-substituted diaryl derivative or physiologically compatible salt thereof with an organic or inorganic acid according to formula II

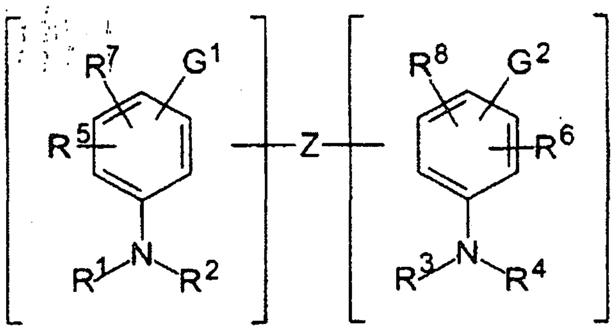

(II)

in which

$R^1$, $R^2$, $R^3$ and $R^4$ independently of one another are a bond to the bridge Z, a hydrogen atom, a ($C_1$-$C_6$)-alkyl group, a ($C_2$-$C_6$)-alkenyl group, an optionally substituted aryl group, a ($C_1$-$C_6$)-alkoxy- ($C_1$-$C_6$)-alkyl group, a ($C_1$-$C_6$)-hydroxyalkyl group or an aryl-($C_1$-$C_6$)-alkyl group, where also in each case the radicals $R^1$ and $R^2$ and/or $R^3$ and $R^4$, together with the nitrogen atom, can form a 5- or 6-membered ring,
$R^5$, $R^6$, $R^7$ and $R^8$ independently of one another are a hydrogen atom, a halogen atom, a ($C_1$-$C_6$)-alkyl group, a ($C_1$-$C_6$)-alkoxy group, a ($C_1$-$C_6$)-hydroxyalkoxy group, a ($C_2$-$C_6$)-polyhydroxyalkyl group, a ($C_1$-$C_6$)-alkoxy-($C_1$-$C_6$)-alkyl group, a hydroxy-($C_1$-$C_6$)-alkoxy group, a hydroxy group, a cyano group, a nitro group, a carboxy group, a sulphonic acid group, a sulphamoyl group, an optionally substituted arylazo group or a group $R^IR^{II}N$-$(CH_2)_{n}$-, in which $R^I$ and $R^{II}$, independently of one another, are a hydrogen atom, a ($C_1$-$C_6$)-alkyl group, a ($C_2$-$C_6$)-alkenyl group, an optionally substituted aryl group, a ($C_1$-$C_6$) -alkoxy-($C_1$-$C_6$)-alkyl group, a ($C_1$-$C_6$)-hydroxyalkyl group or an aryl-($C_1$-$C_6$)-alkyl group, where $R^I$ and $R^{II}$, together with the nitrogen atom, can form a 5- or 6-membered heterocycle, and n is a number 0, 1, 2, 3 or 4,
$G^1$ and $G^2$ independently of one another are a hydroxy group or a group -$NR^9R^{10}$, in which $R^9$ and $R^{10}$, independently of one another, are a hydrogen atom, a ($C_1$-$C_6$)-alkyl group, a ($C_1$-$C_6$) -hydroxyalkyl group or a bond to the bridge Z,
Z is a direct bond, a group NR‴, in which R‴ is a hydrogen atom or a ($C_1$-$C_6$)-alkyl group, a sulphur atom, a carbonyl group, a vinylene group, an alkylene group having 1 to 14 carbon atoms, such as, for example, a linear or branched alkylene chain or an alkylene ring, where these can be interrupted or terminated by one or more nitrogen-containing groups and/or one or more heteroatoms, such as oxygen, sulphur or nitrogen atoms, and can possibly be substituted by one or more hydroxyl or $C_1$- to $C_8$-alkoxy radicals,

with the provisos that the compounds of the formula II contain only one bridge Z per molecule and the compounds of the formula II contain at least one amino group which carries at least one hydrogen atom, and
(B2) at least one primary or secondary aromatic amine different from (B1), or physiologically compatible salt with an organic or inorganic acid.

2. Composition according to Claim 1, **characterized in that**, in formula I, the group -CHO is bonded in the 2- or 4-position.

3. Composition according to one of Claims 1 or 2, **characterized in that** the compounds according to formula I are selected from the group consisting of the benzenesulphonates, p-toluenesulphonates, methanesulphonates, trifluoromethanesulphonates, perchlorates, sulphates, chlorides, bromides, iodides and/or tetrachlorozincates of 4-formyl-1-methylquinolinium and 2-formyl-1-methylquinolinium.

**4.** Composition according to one of Claims 1 to 3, **characterized in that** component A is present as hydrate.

**5.** Composition according to one of Claims 1 to 4, **characterized in that** the compounds of component B1 are selected from the group which is formed from 4,4'-diaminodiphenylamine, N,N'-bis(2-hydroxyethyl)-N,N'-bis(4-aminophenyl)-1,3-diaminopropan-2-ol, N,N'-bis(4-aminophenyl)-1,4-diazacycloheptane, bis(5-amino-2-hydroxyphenyl)methane, 1,3-bis(2,4-diaminophenoxy)propane, 1,8-bis(2,5-diaminophenoxy)-3,6-dioxaoctane, and the physiologically compatible salts of these compounds.

**6.** Composition according to one of Claims 1 to 5, **characterized in that** component B2 is selected from compounds according to formula III

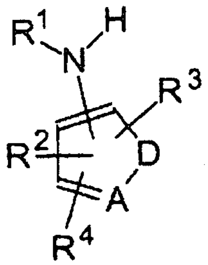

(III)

in which

A is a group C-$R^5$, in which $R^5$ is a hydrogen atom or a bond to one of the radicals $R^2$, $R^3$ or $R^4$, or is a nitrogen atom,
D is an optionally substituted vinylene group, a group -N=C$R^6$-, in which $R^6$ is a hydrogen atom or a bond to one of the radicals $R^2$, $R^3$ or $R^4$, or is a group N-$R^7$, in which $R^7$ is a ($C_1$-$C_6$)-alkyl group, a ($C_2$-$C_6$)-alkenyl group, a ($C_1$-$C_6$)-hydroxyalkyl group, a ($C_2$-$C_6$)-polyhydroxyalkyl group or an aryl-($C_1$-$C_6$)-alkyl group,
$R^1$ is a hydrogen atom, a ($C_1$-$C_6$)-alkyl group, a ($C_2$-$C_6$)-alkenyl group, a ($C_1$-$C_6$) -hydroxyalkyl group, a ($C_2$-$C_6$)-polyhydroxyalkyl group, an aryl-($C_1$-$C_6$)-alkyl group, where $R^1$, together with the remainder of the molecule, can form a fused 5- or 6-membered ring,
$R^2$, $R^3$ and $R^4$ independently of one another are a hydrogen atom, a halogen atom, a ($C_1$-$C_6$)-alkyl group, a ($C_1$-$C_6$)-alkoxy group, a ($C_1$-$C_6$)-hydroxyalkoxy group, a ($C_2$-$C_6$)-polyhydroxyalkyl group, a ($C_1$-$C_6$)-alkoxy-($C_1$-$C_6$)-alkyl group, a hydroxy-($C_1$-$C_6$)-alkoxy group, a hydroxy group, a cyano group, a nitro group, a carboxy group, a sulphonic acid group, a sulphamoyl group, an optionally substituted arylazo group, a group $R^IR^{II}N$- $(CH_2)_n$-,
in which $R^I$ and $R^{II}$, independently of one another, are a hydrogen atom, a ($C_1$-$C_6$)-alkyl group, a ($C_2$-$C_6$)-alkenyl group, an optionally substituted aryl group, a ($C_1$-$C_6$) -alkoxy- ($C_1$-$C_6$) -alkyl group, a ($C_1$-$C_6$)-hydroxyalkyl group or an aryl-($C_1$-$C_6$)-alkyl group, where $R^I$ and $R^{II}$, together with the nitrogen atom, can form a 5- or 6-membered heterocycle, and n is a number 0, 1, 2, 3 or 4,
where also two of the radicals $R^2$, $R^3$ and $R^4$ can together form a fused 5- or 6-membered carbocyclic or heterocyclic, aliphatic or aromatic ring
or physiologically compatible salts thereof with inorganic or organic acids.

**7.** Composition according to one of Claims 1 to 5, **characterized in that** the compounds of component B2 are selected from the group consisting of N,N-bis(2-hydroxyethyl)-p-phenylenediamine, 1-(2-hydroxyethyl)-p-phenylenediamine, 2,5-diaminotoluene, 2-(2,4-diaminophenoxy)ethanol, 2-amino-4-(2-hydroxyethyl)aminoanisole, 4-aminophenol, 4-amino-3-methylphenol, 4-amino-2,6-dichlorophenol, 2-aminophenol, 3-aminophenol, 5-amino-2-methylphenol, 3-amino-2-chloro-6-methylphenol, 2-amino-6-chloro-4-nitrophenol, 2,4,5,6-tetraaminopyrimidine, 4-hydroxy-2,5,6-triaminopyrimidine, 2-amino-3-hydroxypyridine, 3-amino-2-methylamino-6-methoxypyridine, 3,5-diamino-2,6-dimethoxypyridine, 4,5-diamino-1-(2-hydroxyethyl)pyrazole, and the physiologically compatible salts of said compounds.

**8.** Composition according to one of Claims 1 to 7, **characterized in that** it additionally comprises linear or branched, aliphatic polyols.

**9.** Composition according to one of Claims 1 to 8, **characterized in that** it additionally comprises at least one polymer selected from anionic, cationic, nonionic and amphoteric polymers.

**10.** Composition according to one of Claims 1 to 9, **characterized in that** it additionally comprises at least one surfactant selected from anionic, cationic, nonionic, amphoteric and zwitterionic surfactants.

**11.** Composition according to one of Claims 1 to 10, **characterized in that** it additionally comprises at least one fatty alcohol having 6 to 30 carbon atoms.

**12.** Method of colouring keratin-containing fibres, in particular human hair, **characterized in that** a composition according to one of Claims 1 to 11 is applied to the fibres and rinsed out again after a contact time.

**Revendications**

**1.** Agent pour la teinture de fibres kératiniques, en particulier de cheveux humains, qui contient une combinaison des composants

(A) au moins un dérivé formylé de quinolinium répondant à la formule I, ainsi que leurs dérivés

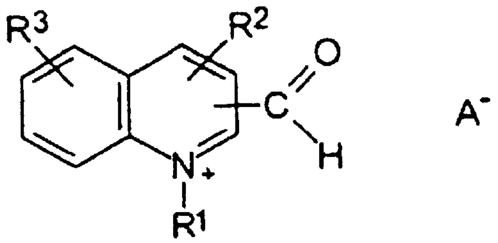

(I)

dans laquelle

$R^1$ représente un groupe alkyle en $C_1$-$C_6$, un groupe alcényle en $C_2$-$C_6$, un groupe aryle, un groupe arylalkyle en $C_1$-$C_6$ ou un groupe hétéroarylalkyle en $C_1$-$C_6$,
$R^2$ et $R^3$ représentent, indépendamment l'un de l'autre, respectivement un atome d'hydrogène, un atome d'halogène, un groupe alkyle en $C_1$-$C_6$, un groupe alcoxy en $C_1$-$C_6$, un groupe hydroxyalcoxy en $C_1$-$C_6$, un groupe hydroxyle, un groupe nitro, un groupe aryle, un groupe trifluorométhyle, un groupe amino qui peut être substitué par des groupes alkyle en $C_1$-$C_6$, ou un groupe acyle en $C_1$-$C_6$,
$A^-$ représente un anion physiologiquement acceptable ou un N-oxyde de la quinoléine,

(B1) au moins un dérivé de diaryle substitué par un ou plusieurs groupes amino, ou son sel physiologiquement acceptable avec un acide organique ou inorganique, répondant à la formule II

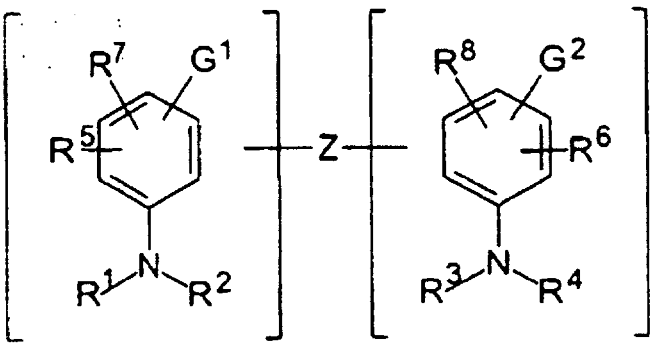

(II)

dans laquelle

$R^1$, $R^2$, $R^3$ et $R^4$ représentent, indépendamment les uns des autres, une liaison pour le pontage Z, un atome

d'hydrogène, un groupe alkyle en $C_1$-$C_6$, un groupe alcényle en $C_2$-$C_6$, un groupe aryle éventuellement substitué, un groupe alcoxy(en $C_1$-$C_6$)alkyle en $C_1$-$C_6$, un groupe hydroxyalkyle en $C_1$-$C_6$ ou un groupe arylalkyle en $C_1$-$C_6$, respectivement les radicaux $R^1$ et $R^2$ et/ou $R^3$ et $R^4$, de manière conjointe avec l'atome d'azote, pouvant également former un noyau à 5 ou à 6 membres ;
$R^5$, $R^6$, $R^7$ et $R^8$ représentent, indépendamment les uns des autres, un atome d'hydrogène, un atome d'halogène, un groupe alkyle en $C_1$-$C_6$, un groupe alcoxy en $C_1$-$C_6$, un groupe hydroxyalcoxy en $C_1$-$C_6$, un groupe polyhydroxyalkyle en $C_2$-$C_6$, un groupe alcoxy(en $C_1$-$C_6$)alkyle en $C_1$-$C_6$, un groupe hydroxyalcoxy en $C_1$-$C_6$, un groupe hydroxyle, un groupe cyano, un groupe nitro, un groupe carboxyle, un groupe d'acide sulfonique, un groupe sulfamoyle, un groupe arylazo éventuellement substitué, ou un groupe $R^I$-$N^{II}$-$(CH_2)_n$-,
dans lequel $R^I$ et $R^{II}$ représentent, indépendamment l'un de l'autre, un atome d'hydrogène, un groupe alkyle en $C_1$-$C_6$, un groupe alcényle en $C_2$-$C_6$, un groupe aryle éventuellement substitué, un groupe alcoxy(en $C_1$-$C_6$)alkyle en $C_1$-$C_6$, un groupe hydroxyalkyle en $C_1$-$C_6$ ou un groupe arylalkyle en $C_1$-$C_6$, les radicaux $R^1$ et $R^{II}$, de manière conjointe avec l'atome d'azote, pouvant également former un hétérocycle à 5 ou à 6 membres, et n représente le nombre 0, 1, 2, 3 ou 4 ;
$G^1$ et $G^2$ représentent, indépendamment l'un de l'autre, un groupe hydroxyle ou un groupe $-NR^9R^{10}$ dans lequel $R^9$ et $R^{10}$ représentent, indépendamment l'un de l'autre, un atome d'hydrogène, un groupe alkyle en $C_1$-$C_6$, un groupe hydroxyalkyle en $C_1$-$C_6$ ou une liaison pour le pontage Z ;
Z représente une liaison directe, un groupe NR''' dans lequel R''' représente un atome d'hydrogène ou un groupe alkyle en $C_1$-$C_6$, un atome de soufre, un groupe carbonyle, un groupe vinylène, un groupe alkylène contenant de 1 à 14 atomes de carbone, comme par exemple une chaîne alkylène linéaire ou ramifiée ou un noyau alkylène, qui peuvent être interrompus ou terminés par un ou plusieurs groupes azotés et/ou un ou plusieurs hétéroatomes tels qu'un atome d'oxygène, un atome de soufre ou un atome d'azote et qui peuvent éventuellement être substitués par un ou plusieurs radicaux hydroxyle ou alcoxy en $C_1$-$C_8$;

avec les mesures que les composés répondant à la formule II ne contiennent qu'un seul pontage Z par molécule et que les composés répondant à la formule II contiennent au moins un groupe amino qui porte au moins un atome d'hydrogène ; et
(B2) au moins une amine aromatique primaire ou secondaire ou un de ses sels physiologiquement acceptables avec un acide organique ou inorganique, différent(e) de (B1).

2. Agent selon la revendication 1, **caractérisé en ce que**, dans la formule I, le groupe -CHO est lié en position 2 ou en position 4.

3. Agent selon l'une quelconque des revendications 1 ou 2, **caractérisé en ce que** les composés répondant à la formule I sont choisis parmi le groupe constitué par les benzènesulfonates, les p-toluènesulfonates, les méthanesulfonates, les trifluorométhanesulfonates, les perchlorates, les sulfates, les chlorures, les bromures, les iodures et/ou les tétrachlorozincates du 4-formyl-1-méthylquinolinium et du 2-formyl-1-méthylquinolinium.

4. Agent selon l'une quelconque des revendications 1 à 3, **caractérisé en ce que** le composant A est présent sous forme d'hydrate.

5. Agent selon l'une quelconque des revendications 1 à 4, **caractérisé en ce que** les composés du composant B1 sont choisis parmi le groupe qui est formé par la 4,4'-diaminodiphénylamine, le N,N'-bis-(2-hydroxyéthyl)-N,N'-bis-(4-aminophényl)-1,3-diamino-propan-2-ol, le N,N'-bis-(4-aminophényl)-1,4-diazacycloheptane, le bis-(5-amino-2-hydroxyphényl)-méthane, le 1,3-bis-(2,4-diaminophénoxy)-propane, le 1,8-bis-(2',5'-diaminophénoxy)-3,6-dioxaoctane, ainsi que les sels physiologiquement acceptables de ces composés.

6. Agent selon l'une quelconque des revendications 1 à 5, **caractérisé en ce que** le composant B2 est choisi parmi des composés répondant à la formule III

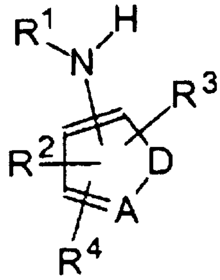

(III)

dans laquelle

A représente un groupe C-$R^5$ dans lequel $R^5$ représente un atome d'hydrogène ou une liaison à un des radicaux $R^2$, $R^3$ ou $R^4$, ou représente un atome d'azote,
D représente un groupe vinylène, éventuellement substitué, un groupe -N=C-$R^6$ dans lequel $R^6$ représente un atome d'hydrogène ou une liaison à un des radicaux $R^2$, $R^3$ ou $R^4$, ou représente un groupe N-$R^7$ dans lequel $R^7$ représente un groupe alkyle en $C_1$-$C_6$, un groupe alcényle en $C_2$-$C_6$, un groupe hydroxyalkyle en $C_1$-$C_6$, un groupe polyhydroxyalkyle en $C_2$-$C_6$ ou un groupe arylalkyle en $C_1$-$C_6$;
$R^1$ représente un atome d'hydrogène, un groupe alkyle en $C_1$-$C_6$, un groupe alcényle en $C_2$-$C_6$, un groupe hydroxyalkyle en $C_1$-$C_6$, un groupe polyhydroxyalkyle en $C_2$-$C_6$, un groupe arylalkyle en $C_1$-$C_6$, $R^1$ pouvant former, conjointement avec la molécule résiduelle, un noyau condensé à 5 membres ou à 6 membres,
$R^2$, $R^3$ et $R^4$ représentent, indépendamment les uns des autres, un atome d'hydrogène, un atome d'halogène; un groupe alkyle en $C_1$-$C_6$, un groupe alcoxy en $C_1$-$C_6$, un groupe hydroxyalcoxy en $C_1$-$C_6$, un groupe polyhydroxyalkyle en $C_2$-$C_6$, un groupe alcoxy(en $C_1$-$C_6$)alkyle en $C_1$-$C_6$, un groupe hydroxyalcoxy en $C_1$-$C_6$, un groupe hydroxyle, un groupe cyano, un groupe nitro, un groupe carboxyle, un groupe d'acide sulfonique, un groupe sulfamoyle, un groupe arylazo éventuellement substitué, un groupe $R^IR^{II}N-(CH_2)_n-$,
dans lequel $R^I$ et $R^{II}$ représentent, indépendamment l'un de l'autre, un atome d'hydrogène, un groupe alkyle en $C_1$-$C_6$, un groupe alcényle en $C_2$-$C_6$, un groupe aryle éventuellement substitué, un groupe alcoxy(en $C_1$-$C_6$) alkyle en $C_1$-$C_6$, un groupe hydroxyalkyle en $C_1$-$C_6$ ou un groupe arylalkyle en $C_1$-$C_6$, les radicaux $R^I$ et $R^{II}$, de manière conjointe avec l'atome d'azote, pouvant également former un hétérocycle à 5 ou à 6 membres, et n représente le nombre 0, 1, 2, 3 ou 4 ;
deux des radicaux $R^2$, $R^3$ et $R^4$ pouvant également former ensemble un noyau aliphatique ou aromatique, carbocyclique ou hétérocyclique, condensé à 5 ou à 6 membres

ou leurs sels physiologiquement acceptables avec des acides inorganiques ou organiques.

**7.** Agent selon l'une quelconque des revendications 1 à 5, **caractérisé en ce que** les composés du composant B2 sont choisis parmi le groupe qui est formé par la N,N-bis-(2-hydroxyéthyl)-p-phénylènediamine, la 1-(2-hydroxyéthyl)-p-phénylènediamine, le 2,5-diaminotoluène, le 2-(2,4-diaminophénoxy)éthanol, le 2-amino-4-(2-hydroxyéthyl) amino-anisole, le 4-aminophénol, le 4-amino-3-méthylphénol, le 4-amino-2,6-dichlorophénol, le 2-aminophénol, le 3-aminophénol, le 5-amino-2-méthylphénol, le 3-amino-2-chloro-6-méthylphénol, le 2-amino-6-chloro-4-nitrophénol, la 2,4,5,6-tétraaminopyrimidine, la 4-hydroxy-2,5,6-triaminopyrimidine, la 2-amino-3-hydroxypyridine, la 3-amino-2-méthylamino-6-méthoxypyridine, la 3,5-diamino-2,6-diméthoxypyridine, le 4,5-diamino-1-(2-hydroxyéthyl)pyrazole, ainsi que les sels physiologiquement acceptables des composés mentionnés.

**8.** Composition selon l'une quelconque des revendications 1 à 7, **caractérisée en ce qu'**elle contient en outre des polyols aliphatiques, linéaires ou ramifiés.

**9.** Agent selon l'une quelconque des revendications 1 à 8, **caractérisé en ce qu'**il contient en outre au moins un polymère choisi parmi des polymères anioniques, cationiques, non ioniques et amphotères.

**10.** Agent selon l'une quelconque des revendications 1 à 9, **caractérisé en ce qu'**il contient en outre au moins un agent tensioactif choisi parmi des agents tensioactifs anioniques, cationiques, non ioniques, amphotères et zwitterioniques.

**11.** Agent selon l'une quelconque des revendications 1 à 10, **caractérisé en ce qu'**il contient en outre au moins un

alcool gras contenant de 6 à 30 atomes de carbone.

**12.** Procédé pour la teinture de fibres kératiniques, en particulier de cheveux humains, **caractérisé en ce qu'**on applique sur les fibres un agent selon l'une quelconque des revendications 1 à 11, que l'on fait disparaître par rinçage après l'avoir laissé agir pendant un certain temps.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9918916 A1
- WO 0038638 A1
- WO 0134106 A1
- WO 0147483 A1
- EP 998908 A2
- GB 2104091 A
- EP 47714 A
- EP 217274 A
- EP 283817 A
- DE 2817369
- DE 3929973
- DE 4413686 C
- DE 4440625 A1
- DE 19503465 A1
- DE 3725030 A
- DE 3723354 A
- DE 3926344 A


### In der Beschreibung aufgeführte Nicht-Patentliteratur


- Dermatology. Verlag Marcel Dekker Inc, 1986, vol. 7
- **CH. ZVIAK.** The Science of Hair Care. vol. 7, 248-250
- THE SCIENCE OF HAIR CARE. vol. 8, 264-267